(19) Europäisches Patentamt
European Patent Office
Office européen des brevets

(11) EP 0 564 406 B1

(12) EUROPÄISCHE PATENTSCHRIFT

(45) Veröffentlichungstag und Bekanntmachung des Hinweises auf die Patenterteilung:
**06.05.1999 Patentblatt 1999/18**

(51) Int. Cl.[6]: **C07F 17/02**, B01J 31/28, C07B 31/00, C07B 53/00

(21) Anmeldenummer: **93810212.6**

(22) Anmeldetag: **24.03.1993**

# (54) Ferrocenyldiphosphine als Liganden für homogene Katalysatoren

Ferrocenyldiphosphine as ligands for homogeneous catalysts

Ferrocenyldiphosphine comme ligandes de catalyseurs homogènes

(84) Benannte Vertragsstaaten:
**BE CH DE FR GB IT LI NL**

(30) Priorität: **02.04.1992 CH 1068/92**

(43) Veröffentlichungstag der Anmeldung:
**06.10.1993 Patentblatt 1993/40**

(73) Patentinhaber: **Novartis AG**
**4058 Basel (CH)**

(72) Erfinder:
- **Togni, Antonio, Dr.**
  **CH-8966 Oberwil-Lieli (CH)**
- **Spindler, Felix, Dr.**
  **CH-4656 Starrkirch-Wil (CH)**
- **Zanetti, Nadia**
  **CH-8049 Zürich (CH)**
- **Tijani, Amina, Dr.**
  **F-68520 Burnhaupt-le-Haut (FR)**

(56) Entgegenhaltungen:
- **BULLETIN OF THE CHEMICAL SOCIETY OF JAPAN Bd. 53, 1980, Seiten 1138 - 1151 HYASHI, T. ET AL.**
- **TETRAHEDRON LETTERS Nr. 14, 1976, Seiten 1133 - 1134 HAYASHI, T. ET AL.**
- **Schreiben The British Library, 12.04.96**
- **Doktorarbeit von K. Baker, Universität Oxford, Seiten 68-80, veröffentlicht spätestens am 4/12/ 1991**
- **Schreiben von Prof. John M. Brown, Universität Oxford vom 18/5/94**
- **Schreiben The British Library, 12.04.96**

Bemerkungen:
Die Akte enthält technische Angaben, die nach dem Eingang der Anmeldung eingereicht wurden und die nicht in dieser Patentschrift enthalten sind.

EP 0 564 406 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Xerox (UK) Business Services
2.16.7/3.6

## Beschreibung

Die Erfindung betrifft 1-[2-(Diphenylphosphino)ferrocenyl]alkylidenphosphine in Form von Racematen und Stereoisomeren, ein Verfahren zu deren Herstellung, Iridium-und Rhodium-Komplexe mit diesen Liganden und deren Verwendung als enantioselektive Hydrierkatalysatoren für die homogene Hydrierung von prochiralen ungesättigten Verbindungen.

T. Hayashi et al. beschreiben im Bull. Chem. Soc. Jpn., 53, Seiten 1136-1151 die Herstellung von einem chiralen Ferrocenylphosphin als Ligand für Übergangsmetallkomplexe zur asymmetrischen Synthese, nämlich [(R)-[(S)-2-(Diphenylphosphino)ferrocenyl]ethyl]diphenylphosphin. Eigene Untersuchungen ergaben, dass homogene Hydrierungen von prochiralen Verbindungen mit Rhodium-Komplexen, die diesen Liganden enthalten, nur niedrige optische Ausbeuten ergeben.

Es wurde nun gefunden, dass bei gleichen oder sogar kürzeren Reaktionszeiten die Enantioselektivität ganz wesentlich gesteigert werden kann, wenn die Substituenten in der Alkylidenphosphingruppe nicht beide Phenyl darstellen.

Ein Gegenstand der Erfindung sind Verbindungen der Formel I,

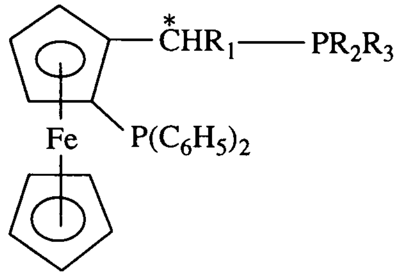

(I),

worin $R_1$ $C_1$-$C_8$-Alkyl, Phenyl oder mit 1 bis 3 $C_1$-$C_4$-Alkyl oder $C_1$-$C_4$-Alkoxy substituiertes Phenyl bedeuten; $R_2$ und $R_3$ gleich sind und $C_1$-$C_{12}$-Alkyl, $C_5$-$C_{12}$-Cycloalkyl, 4-Trifluoromethyl im Falle $R_1$ gleich Methyl, mit $C_1$-$C_4$-Alkyl oder $C_1$-$C_4$-Alkoxy substituiertes $C_5$-$C_{12}$-Cycloalkyl oder mit ein bis drei $C_1$-$C_4$-Alkyl, $C_1$-$C_4$-Alkoxy, $-SiR_4R_5R_6$, Halogen, $-SO_3M$, $-CO_2M$, $-PO_3M$, $-NR_7R_8$ oder $-[^{\oplus}NR_7R_8R_9]X^{\ominus}$ substituiertes Phenyl darstellen; oder $R_2$ und $R_3$ verschieden sind und $C_1$-$C_{12}$-Alkyl, $C_5$-$C_{12}$-Cycloalkyl, mit $C_1$-$C_4$-Alkyl oder $C_1$-$C_4$-Alkoxy substituiertes $C_5$-$C_{12}$-Cycloalkyl, Phenyl oder mit ein bis drei $C_1$-$C_4$-Alkyl, $C_1$-$C_4$-Alkoxy, $-SiR_4R_5R_6$, Halogen, $-SO_3M$, $-CO_2M$, $-PO_3M$, $-NR_7R_8$ oder $-[^{\oplus}NR_7R_8R_9]X^{\ominus}$ substituiertes Phenyl bedeuten; oder die Gruppe $-PR_2R_3$ einen Rest der Formel II

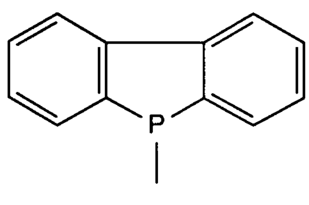

(II)

darstellt, und $R_4$, $R_5$ und $R_6$ unabhängig voneinander für $C_1$-$C_{12}$-Alkyl oder Phenyl stehen, $R_7$ und $R_8$ H, $C_1$-$C_{12}$-Alkyl, Phenyl oder $R_7$ und $R_8$ zusammen Tetramethylen, Pentamethylen oder 3-Oxa-1,5-Pentylen darstellen, $R_9$ H oder $C_1$-$C_4$-Alkyl bedeutet, M für H oder ein Alkalimetall steht, $X^{\ominus}$ das Anion einer einbasischen Säure ist, und * ein stereogenes C-Atom kennzeichnet, in Form Ihrer Racemate und Diastereomeren oder Gemischen von Diastereomeren, ausgenommen Verbindungen der Formel I, worin $R_1$ Methyl ist und die Gruppe $-PR_2R_3$ einen Rest der Formel II oder $P(cyclohexyl)_2$ bedeutet.

$R_1$ in der Bedeutung von Alkyl kann linear oder verzweigt sein und es enthält bevorzugt 1 bis 4 C-Atome. Beispiele für dieses Alkyl sind Methyl, Ethyl, n- und i-Propyl, n-, i- und t-Butyl, Pentyl, Hexyl, Heptyl und Octyl. Bevorzugt sind Methyl und Ethyl und besonders bevorzugt ist Methyl.

$R_1$ enthält als substituiertes Phenyl bevorzugt 1 oder 2 Substituenten. Alkylsubstituenten können zum Beispiel Methyl, Ethyl, n- und i-Propyl, n-, i- und t-Butyl sein; bevorzugt sind Methyl und Ethyl. Alkoxysubstituenten können zum Beispiel Methoxy, Ethoxy, n-und i-Propoxy, n-, i- und t-Butoxy sein; bevorzugt sind Methoxy und Ethoxy.

$R_2$ und $R_3$ in der Bedeutung von Alkyl können linear oder verzweigt sein und sie enthalten bevorzugt 1 bis 8 und besonders bevorzugt 1 bis 4 C-Atome. Beispiele für dieses Alkyl sind Methyl, Ethyl, n- und i-Propyl, n-, i- und t-Butyl, Pentyl, Hexyl, Heptyl, Octyl, Nonyl, Decyl, Undecyl und Dodecyl. Bevorzugt sind Methyl, Ethyl, n- und i-Propyl, n-, i- und t-Butyl. Wenn $R_2$ und $R_3$ gleich sind, bedeuten sie als Alkyl besonders bevorzugt i-Propyl oder t-Butyl.

$R_2$ und $R_3$ in der Bedeutung von Cycloalkyl enthalten bevorzugt 5 bis 8, besonders bevorzugt 5 oder 6 Ring-C-Atome. Beispiele für Cycloalkyl sind Cyclopentyl, Cyclohexyl, Cycloheptyl, Cyclooctyl, Cyclodecyl und Cyclododecyl. Bevorzugt sind Cyclopentyl und Cyclohexyl und besonders bevorzugt ist Cyclohexyl.

Das Cycloalkyl kann substituiert sein, zum Beispiel mit 1 bis 3 Alkyl-oder Alkoxy-Substituenten, Beispiele für solche Substituenten sind zuvor angegeben worden. Bevorzugt sind Methyl und Ethyl sowie Methoxy und Ethoxy. Beispiele für substituiertes Cyclohexyl sind Methyl- und Methoxycyclopentyl und -cyclohexyl.

$R_2$ und $R_3$ in der Bedeutung von substituiertem Phenyl enthält bevorzugt 1 oder 2 Substituenten. Sofern das Phenyl 2 oder 3 Substituenten enthält, können diese gleich oder verschieden sein.

Beispiele für die Substituenten Alkyl und Alkoxy sind zuvor angegeben worden; bevorzugte Alkyl- und Alkoxysubstituenten für Phenyl sind Methyl, Ethyl sowie Methoxy und Ethoxy.

Wenn der Phenyl-Substituent Halogen bedeutet, so handelt es sich bevorzugt um - F, -Cl und -Br.

$R_4$, $R_5$ und $R_6$ können lineares oder verzweigtes Alkyl sein, das bevorzugt 1 bis 8 und besonders bevorzugt 1 bis 4 C-Atome enthält. Beispiele für Alkyl sind zuvor angegeben worden. Bevorzugtes Alkyl ist Methyl, Ethyl, n-Propyl, n-Butyl und t-Butyl. Besonders bevorzugt steht der Substituent $-SiR_4R_5R_6$ für Trimethylsilyl.

Unter den sauren Phenyl-Substituenten $-SO_3M$, $-CO_2M$ und $-PO_3M$ ist die Gruppe $-SO_3M$ bevorzugt. M steht bevorzugt für H, Li, Na und K.

$R_7$ und $R_8$ enthalten als Alkyl bevorzugt 1 bis 6, besonders bevorzugt 1 bis 4 C-Atome. Das Alkyl ist bevorzugt linear. Bevorzugte Beispiele sind Methyl, Ethyl, n-Propyl und n-Butyl. $R_9$ stellt als Alkyl bevorzugt Methyl dar.

$X^{\ominus}$ stellt als Anion einer einbasischen Säure bevorzugt $Cl^{\ominus}$, $Br^{\ominus}$ oder das Anion einer Carbonsäure dar, zum Beispiel Formiat, Acetat, Trichloracetat oder Trifluoracetat dar.

Einige Beispiele für substituiertes Phenyl sind 2-Methyl-, 3-Methyl-, 4-Methyl-, 2- oder 4-Ethyl-, 2- oder 4-i-Propyl-, 2- oder 4-t-Butyl-, 2-Methoxy-, 3-Methoxy-, 4-Methoxy-, 2-oder 4-Ethoxy-, 4-Trimethylsilyl-, 2- oder 4-Fluor-, 2,4-Difluor-, 2- oder 4-Chlor-, 2,4-Dichlor-, 2,4-Dimethyl-, 3,5-Dimethyl-, 2-Methoxy-4-methyl-, 3,5-Dimethyl-4-methoxy-, 3,5-Dimethyl-4-(dimethylamino)-, 2- oder 4-Amino-, 2- oder 4-Methylamino-, 2- oder 4-(Dimethylamnio)-, 2- oder 4-$SO_3H$-, 2- oder 4-$SO_3Na$-, 2- oder 4-[$^{\oplus}NH_3Cl^{\ominus}$]-, 3,4,5-Trimethylphen-1-yl oder 2,4,6-Trimethylphen-1-yl.

Wenn $R_2$ und $R_3$ gleich sind, bedeuten $R_2$ und $R_3$ bevorzugt Cyclohexyl, 2-oder 4-Methylphen-1-yl, 2- oder 4-Methoxyphen-1-yl, 2- oder 4-(Dimethylamino)phen-1-yl, 3,5-Dimethyl-4-(dimethylamino)phen-1-yl und 3,5-Dimethyl-4-methoxyphen-1-yl.

Wenn $R_2$ und $R_3$ verschieden sind, bedeuten $R_2$ bevorzugt Phenyl und $R_3$ bevorzugt Cyclohexyl, 2- oder 4-Methylphen-1-yl, 2- oder 4-Methoxyphen-1-yl, 4-(Dimethylamino)phen-1-yl, 3,5-Dimethyl-4-(dimethylamino)phen-1-yl, 3,5-Dimethyl-4-methoxyphen-1-yl oder 4-t-Butyl-phen-1-yl.

In einer bevorzugten Ausführungsform bedeuten $R_2$ und $R_3$ gleiche Reste und bedeuten Cyclohexyl.

In einer anderen bevorzugten Ausführungsform bedeuten $R_2$ und $R_3$ gleiche Reste und stellen tert.-Butyl oder o-Anisyl dar.

In einer weiteren bevorzugten Ausführungsform bedeutet $R_2$ Phenyl und $R_3$ o-Anisyl.

In einer besonders bevorzugten Ausführungsform bedeuten in Formel I $R_1$ Methyl und $R_2$ und $R_3$ je Cyclohexyl.

Ein weiterer Gegenstand der Erfindung ist ein Verfahren zur Herstellung von Verbindungen der der Formel I, das dadurch gekennzeichnet ist, dass man eine Verbindung der Formel III

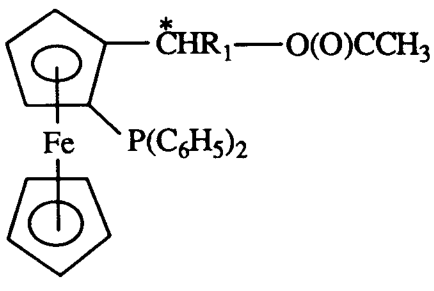

(III)

in Gegenwart eines inerten Lösungsmittels bei Raumtemperatur oder erhöhter Temperatur mit einem Phosphin der Formel IV

$$HPR_2R_3 \qquad (IV)$$

umsetzt.

Die Umsetzung ist an sich bekannt und von T. Hayashi et al. im Bull. Chem. Soc. Jpn., 53, Seiten 1136-1151 beschrieben. Die Herstellung von Verbindungen der Formel III ist dort ebenfalls beschrieben oder kann analog erfolgen. Die Phosphine der Formel IV sind bekannt oder nach bekannten Verfahren in analoger Weise herstellbar.

Die Reaktionstemperatur kann zum Beispiel 20 bis 150 °C, bevorzugt 40 bis 100 °C betragen. Als Lösungsmittel eignen sich polare protische und aprotische Lösungsmittel, die alleine oder in Mischung von zwei oder mehreren Lösungsmitteln verwendet werden können. Einige Beispiele für Lösungsmittel sind Alkanole wie zum Beispiel Methanol und Ethanol, und Carbonsäuren wie zum Beispiel Ameisensäure und Essigsäure.

Die Verbindungen der Formel I werden als Racemate, Gemische von Stereoisomeren oder als Stereoisomere erhalten, je nach dem, ob die Verbindungen der Formel III Racemate, Gemische von Stereoisomeren oder als Stereoisomere eingesetzt werden. Racemate und Gemische von Stereoisomeren können mittels bekannter Methoden in die Stereoisomeren getrennt werden, wobei im allgemeinen chromatographische Methoden bevorzugt werden.

Die Isolierung und Reinigung der Verbindungen der Formel I erfolgt nach an sich bekannten Methoden, zum Beispiel Destillation, Extraktion, Kristallisation und/oder chromatographischen Methoden.

Die erfindungsgemässen Verbindungen der Formel I eignen sich als Liganden für Rhodium-und Iridiumkomplexe. Ein weiterer Gegenstand der Erfindung sind Komplexe der Formeln V und VI,

$$[X_1M_1YZ] \quad (V),$$

$$[X_1M_1Y]^{\oplus}A_1^{\ominus} \quad (VI),$$

worin $X_1$ für zwei $C_2$-$C_{12}$-Olefine oder ein $C_5$-$C_{12}$-Dien steht, Z für Cl, Br oder I steht, $A_1^{\ominus}$ das Anion einer Sauerstoffsäure oder Komplexsäure bedeutet, $M_1$ für Rh oder Ir steht und Y ein erfindungsgemässes Diphosphin der Formel I darstellt, inclusive Verbindungen der Formel I, worin $R_1$ Methyl ist und die Gruppe $-PR_2R_3$ einen Rest der Formel II oder $P(cyclohexyl)_2$ bedeutet. Die Komplexe der Formel VI sind bevorzugt.

Für die Diphosphine der Formel I gelten die gleichen zuvor angeführten Bevorzugungen und beispielhaften Ausführungen. $X_1$ in der Bedeutung als Olefin enthält bevorzugt 2 bis 6 und besonders bevorzugt 2 bis 4 C-Atome. Besonders bevorzugt ist Ethylen; weitere Beispiele sind Propen und 1 -Buten. $X_1$ enthält als Dien bevorzugt 5 bis 8 C-Atome, wobei es sich um ein offenkettiges oder mono- oder bicyclisches Dien handeln kann. Die beiden Olefingruppen des Diens sind bevorzugt durch ein oder zwei $CH_2$-Gruppen verbunden. Beispiele sind 1,3-Pentadien, Cyclopentadien, 1,5-Hexadien, 1,4-Cyclohexadien, 1,4- oder 1,5-Heptadien, 1,4- oder 1,5-Cycloheptadien, 1,4- oder 1,5-Octadien, 1,4- oder 1,5-Cyclooctadien, Norbornadien. Bevorzugt stellt $X_1$ zwei Ethylen, 1,5-Hexadien, 1,5-Cyclooctadien oder Norbornadien dar.

In Formel V steht Z bevorzugt für Cl oder Br. Beispiele für $A_1^{\ominus}$ in Formel VI sind $ClO_4^{\ominus}$, $FSO_3^{\ominus}$, $CH_3SO_3^{\ominus}$, $CF_3SO_3^{\ominus}$, $BF_4^{\ominus}$, $PF_6^{\ominus}$, $SbCl_6^{\ominus}$, $AsF_6^{\ominus}$ und $SbF_6^{\ominus}$. Bevorzugt ist $A_1^{\ominus}$ $ClO_4^{\ominus}$, $CF_3SO_3^{\ominus}$, $BF_4^{\ominus}$, $PF_6^{\ominus}$ und $SbF_6^{\ominus}$.

Die erfindungsgemässen Komplexe werden in an sich bekannter Weise durch die Umsetzung von äquimolaren Mengen einer Verbindung der Formel I mit einem Metallkomplex der Formeln $[M_1(X_1)Z]_2$ oder $M_1(X_1)_2^{\oplus}A_1^{\ominus}$ erhalten, worin $M_1$, $X_1$, Z und $A_1^{\ominus}$ die zuvor angegebenen Bedeutungen haben. Die Metallkomplexe sind bekannt, siehe zum Beispiel EP-A-0 302 021 uns US-A-5 011 995.

Die Umsetzung wird vorteilhaft unter Inertgasatmosphäre, zum Beispiel Argon, und zweckmässig bei Temperaturen von 0 bis 40 °C, bevorzugt bei Raumtemperatur vorgenommen. Vorteilhaft wird ein Lösungsmittel oder Gemisch von Lösungsmitteln mitverwendet, zum Beispiel Kohlenwasserstoffe (Benzol, Toluol, Xylol), halogenierte Kohlenwasserstoffe (Methylenchlorid, Chloroform, Chlorbenzol), Alkanole (Methanol, Ethanol, Ethylenglykolmonomethylether), und Ether (Diethylether, Dibutylether, Ethylenglykoldimethylether, Tetrahydrofuran, Dioxan) oder Gemische davon. Die erfindungsgemässen Komplexe können nach üblichen Methoden isoliert und gereinigt werden, oder sie können vor einer Hydrierung in situ hergestellt und dann in gelöster Form direkt als Hydrierkatalysator eingesetzt werden.

Die erfindungsgemässen Komplexe eignen sich hervorragend als homogene Katalysatoren zur enantioselektiven Hydrierung von prochiralen Verbindungen mit Kohlenstoff-und Kohlenstoff-/Heteroatomdoppelbindungen, zum Beispiel Verbindungen, die eine der Gruppen C=C, C=N, C=O, C=C-N oder C=C-O enthalten [siehe zum Beispiel K. E. König, The Applicability of Asymmetric Homogeneous Catalysis, in James D. Morrison (ed.), Asymmetric Synthesis, Vol. 5, Academic Press, 1985]. Beispiele für solche Verbindungen sind prochirale Olefine, Enamine, Imine und Ketone. Es werden überraschend hohe Ausbeuten, im allgemeinen sogar ein quantitativer chemischer Umsatz, in kurzen Reaktionszeiten erzielt. Besonders überraschend sind die sehr hohen optischen Ausbeuten, die mit den erfindungsgemässen Komplexen erreicht werden. Der Enantiomerenüberschuss (ee) kann über 90 % betragen. Es können hierbei Racemate, Gemische von Stereoisomeren oder Stereoisomere der Komplexe der Formeln V und VI verwendet werden, wobei Gemische von Stereoisomeren oder Stereoisomere bevorzugt sind.

Ein weiterer Gegenstand der Erfindung ist die Verwendung der erfindungsgemässen Komplexe der Formeln V und VI als homogene Katalysatoren zur asymmetrischen Hydrierung von prochiralen Verbindungen mit Kohlenstoff-

oder Kohlenstoff-/Heteroatomdoppelbindungen, besonders mit Gruppen C=C, C=N, C=O, C=C-N oder C=C-O. Bevorzugt ist die Verwendung zur Hydrierung von unsymmetrischen Kohlenstoffdoppelbindungen, Ketiminen und Ketonen. Der erfindungsgemäss als Iridium-Komplex ausgebildete Katalysator der Formeln V und VI wird auch bevorzugt zur Hydrierung von prochiralen N-Arylketiminen zu optisch aktiven sekundären Aminen verwendet. Der erfindungsgemäss als Rhodium-Komplex ausgebildete Katalysator der Formeln V und VI wird bevorzugt zur Hydrierung von Kohlenstoffdoppelbindungen, zum Beispiel prochiralen Kohlenstoffdoppelbindungen verwendet.

Ein anderer Gegenstand der Erfindung ist ein Verfahren zur asymmetrischen Hydrierung von prochiralen Verbindungen mit Kohlenstoff- oder Kohlenstoff-/Heteroatomdoppelbindungen unter homogenen Reaktionsbedingungen, das dadurch gekennzeichnet ist, dass man die Verbindungen bei einer Temperatur von -20 bis 80 °C und einem Wasserstoffdruck von $10^4$ bis $10^7$ Pa in Gegenwart katalytischer Mengen eines Komplexes der Formeln V oder VI hydriert.

Bevorzugte prochirale Verbindungen sind zuvor erwähnt worden. Unsymmetrische Ketimine und Ketone sind bekannt. In Frage kommende N-Arylketimine sind zum Beispiel in der EP-A-0 256 982 beschrieben. N-aliphatische Ketimine sind zum Beispiel in der EP-A-0 301457 beschrieben. Solche Imine können aus den entsprechenden unsymmetrische Ketonen hergestellt werden, die bekannt und teilweise käuflich oder nach bekannten Verfahren herstellbar sind. Geeignete substituierte Alkene sind in der zuvor erwähnten Publikation von K. E. König beschrieben.

Das Verfahren wird bevorzugt bei einer Temperatur von -10 bis 50 °C und bevorzugt bei einem Wasserstoffdruck von $1 \cdot 10^5$ bis $6 \cdot 10^6$ Pa durchgeführt.

Die Menge des Katalysators wird bevorzugt so gewählt, dass das Molverhältnis der zu hydrierenden Verbindung (Substrat) zu dem Komplex der Formeln V oder VI bevorzugt 10000 bis 20, besonders bevorzugt 5000 bis 20, insbesondere bevorzugt 2000 bis 40, und ganz besonders bevorzugt 1000 bis 50 beträgt.

Eine bevorzugte Verfahrensdurchführung ist dadurch gekennzeichnet, dass man zusätzlich ein Ammonium- oder Alkalimetallchlorid, -bromid oder -iodid mitverwendet, besonders bei der Verwendung erfindungsgemässer Iridium-Katalysatoren. Die Menge kann beispielsweise 0,1 bis 100, bevorzugt 1 bis 50 und besonders bevorzugt 2 bis 20 Äquivalente betragen, bezogen auf den Komplex der Formeln V oder VI. Der Zusatz von Iodiden ist bevorzugt. Ammonium ist bevorzugt Tetraalkylammonium mit 1 bis 6 C-Atomen in den Alkylgruppen, und als Alkalimetall ist Lithium, Natrium und Kalium bevorzugt.

Die Hydrierung kann ohne oder in Gegenwart von Lösungsmitteln durchgeführt werden. Geeignete Lösungsmittel, die alleine oder als Mischung von Lösungsmitteln eingesetzt werden können, sind zum Beispiel: Aliphatische und aromatische Kohlenwasserstoffe (Pentan, Hexan, Cyclohexan, Methylcyclohexan, Benzol, Toluol, Xylol), Alkohole wie z.B. Methanol, Ethanol, Propanol und Butanol; Ether wie z.B. Diethylether, Diethylenglykoldimethylether, Ethylenglykoldimethylether, Tetrahydrofuran und Dioxan; Halogenkohlenwasserstoffe wie z.B. Methylenchlorid, Chloroform, 1,1,2,2-Tetrachlorethan und Chlorbenzol; Ester und Lactone wie z.B. Essigsäureethylester, Butyrolacton oder Valerolacton; Carbonsäureamide und Lactame wie z.B. Dimethylformamid, Dimethylacetamid und N-Methylpyrrolidon. Bevorzugte Mischungen sind solche aus Alkoholen und aromatischen Kohlenwasserstoffen, zum Beispiel Methanol/Benzol oder Methanol/Toluol. Bevorzugt wird als Lösungsmittel Methanol alleine oder im Gemisch mit Benzol oder Toluol verwendet.

Mit dem erfindungsgemässen Hydrierverfahren können optisch reine Verbindungen erhalten werden, die wertvolle Zwischenprodukte zur Herstellung biologisch aktiver Wirkstoffe besonders im Bereich der Pharmazeutika und Agrarchemikalien darstellen. So können zum Beispiel aus sekundären Aminen, besonders N-Carbalkoxymethylaminen, 5-Imidazolcarbonsäurederivate hergestellt werden, die eine herbizide Wirkung haben und zur Bekämpfung von Unkräutern verwendet werden können (EP-A-0 207 563). Die optisch reinen $\alpha$-Aminocarbonsäureester sind für Peptidsynthesen geeignet. Aus ungesättigten Aminocarbonsäuren können optisch reine Aminocarbonsäuren erhalten werden, die wertvolle Synthesebausteine darstellen.

Die nachfolgenden Beispiele erläutern die Erfindung näher. Die Reaktionen werden unter Argonatmosphäre durchgeführt. Der chemische Umsatz wird gaschromatographisch bestimmt [Säule DB 17/30 W (15 m), Hersteller: JCW Scientific INC., USA, Temperatur-programm: 60/ 1 min bis 220°C, $\Delta$T: $10° \cdot min^{-1}$]. Die Bestimmung der optischen Ausbeute Enantiomerenüberschuss, ee) erfolgt ebenfalls gaschromatographisch [Säule Chirasil-Val, 50 m, Hersteller. Alltech, USA, T: 150°C, isotherm).

A) Herstellungsbeispiele

Beispiel A1: {(R)-1-[(S)-2-(Diphenylphosphino)ferrocenyl]}ethyl-dicyclohexylphosphin (A).

In einen 25 ml Schlenk-Kolben werden unter Argon-Atmosphäre nacheinander 3,88 g (8,5 mmol) {[(R)-1-[(S)-2-(Diphenylphosphino)ferrocenyl]}ethyl-acetat [(R)-(S)-PPFOAc, hergestellt gemäss Bull. Chem. Soc. Jpn., 53, 1138 (1980)], 60 ml Essigsäure und 1,9 ml (9,35 mmol) Dicyclohexylphosphin eingetragen und anschliessend während 2,5 Stunden unter Rühren auf 50°C erhitzt. Dann werden zu der abgekühlten Reaktionslösung 100 ml Diethylether hinzugefügt. Die Etherphase wird abgetrennt und nacheinander mit Wasser, wässriger Natriumchlorid-Lösung (gesättigt)

und wässriger Natriumbicarbonat-Lösung mehrmals ausgeschüttelt. Die vereinigten Wasser-Phasen werden nocheinmal mit Diethylether ausgeschüttelt. Die Etherphasen werden getrocknet und konzentriert. Nach einer chromatographischen Reinigung über basischem Aluminiumoxid (Lösungsmittel: Hexan/Essigsäureester 9:1) und der anschliessenden Umkristalisation des Rohprodukts aus heissem Ethanol erhält man 4,2 g A (Ausbeute: 81 %) als orange kristalline Substanz.

[a] $^{20}_{D}$: -349 (c = 1,025, $CHCl_3$); Schmelzpunkt: 95-100°C; $^{31}P$-NMR ($CDCl_3$): 15,7 (d,J = 30), -25,8 (d,J = 30).

Beispiel A2: {(R)-1-[(S)-2-Diphenylphosphino)ferrocenyl]}ethyl-di-(2-methoxyphenyl)phosphin (B).

Es wird analog Beispiel A1 verfahren, aber 0,42 g (1,68 mmol) Di-(2-methoxyphenyl)phosphin verwendet. Die Ausbeute beträgt 496 mg B (46%) als orange kristalline Substanz. $^{31}P$-NMR ($CDCl_3$): -21,6 (d, J=11), -25,3 (d, J=11).

Beispiel A3: {($R^*_p$)-(R)-1-[(S)-2-Diphenylphosphino)ferrocenyl]}ethyl-phenyl-(2-methoxyphenyl)phosphin (C).

Es wird analog Beispiel A1 verfahren, aber 0,36 g (1,68 mmol) Phenyl-(2-methoxyphenyl)phosphin verwendet. Die Ausbeute beträgt 555 mg C (54%) als orange kristalline Substanz. $^{31}P$-NMR ($CDCl_3$): -6,1 (d, J=16), -25,5 (d, J=16) (1. Stereoisomer); -7,9 (d, J=14), -25,1 (d, J=14) (2. Stereoisomer).

Beispiel A4: [Norbornadien-{(R)-1-[(S)-2-Diphenylphosphino)ferrocenyl]}ethyl-dicyclohexylphosphinorhodiumtetrafluoroborat] (D).

In einem 25 ml Schlenk-Kolben werden unter Argon-Atmosphäre 100 mg (0,267 mmol) Bis-(Norbornadien)rhodiumtetrafluoroborat in 10 ml des Lösungsmittelgemisches Methanol/Methylenchlorid (1:1) gelöst und danach 159 mg (0,267 mmol) A dazugegeben. Dieses Reaktionsgemisch wird während 60 Minuten bei Raumtemperatur gerührt und anschliessend das Lösungsmittel am Hochvakuum entfernt. Das Rohprodukt wird in 4 ml Methylenchlorid gelöst und durch Fällung mit 15 ml Diethylether erhält man 192 mg orange kristalline Substanz (D) (82%). $^{31}P$-NMR ($CDCl_3$): 23,1 ($J_{RhP}$ 153, $J_{PP}$=36), 52,6 ($J_{RhP}$= 154, $J_{PP}$=36).

Beispiel A5: {(R)-1-[(S)-2-(Diphenylphosphino)ferrocenyl]}ethyl-di-tert.-butylphosphin (**E**)

Es wird analog Beispiel A1 verfahren, aber 1 g (R)-(S)-PPFOAc (2,2 mmol) und 0,37 g Di-tert.-butylphosphin (2,5 mmol) verwendet. Die Ausbeute beträgt 704 mg **E** (59%) als orange kristalline Substanz. $^{31}P$-NMR ($CDCl_3$): 49,9 (d, J=50), -26,1 (d, J=50).

Beispiel A6: {(R)-1-[(S)-2-(Diphenylphosphino)ferrocenyl]}ethyl-di-(2,4,6-trimethylphenyl)phosphin (**F**).

Es wird analog Beispiel A1 verfahren, aber 0,5 g (R)-(S)-PPFOAc (1,1 mmol) und 0,37 g Di-(2,4,6-trimethylphenyl)phosphin (13,3 mmol) verwendet. Die Ausbeute beträgt 313 mg **E** (47%) als orange kristalline Substanz. $^{31}P$-NMR ($CDCl_3$): -6,8 (d, J=17), - 25,0 (d, J=17).

Beispiel A7: {(R)-1-[(S)-2-(Diphenylphosphino)ferrocenyl]}ethyl-di-(4-methoxyphenyl)phosphin (**G**).

Es wird analog Beispiel A1 verfahren, aber 0,548 g (1,2 mmol) (R)-(S)-PPFOAc und 0,29 ml Di-(4-methoxyphenyl)phosphin (1,3 mmol) verwendet. Die Ausbeute beträgt 0,3 g (39%) als oranger Schaum. $^{31}P$-NMR ($CDCl_3$): 2,5 (d, J=16), -25,5 (d, J=16).

Beispiel A8: {($R_p$)-(R)-1-[(S)-2-(Diphenylphosphino)ferrocenyl]}ethyl-(2-methoxyphenyl)phenylphosphin (**H**).

Zu einer Suspension von 1,325 g [$Pd_2Cl_2$((R)-(+)-$C_6H_4CHCH_3N(CH_3)_2)_2$] (2,3 mmol) in 25 ml Methanol werden 2,793 g des Diastereomerengemisches **C** (4,6 mmol) eingetragen. Die resultierende rote Lösung wird während 30 min. bei Raumtemperatur gerührt und anschliessend filtriert. Dann wird eine Lösung von 0,372 $NH_4PF_6$ (2,3 mmol) in 6 ml $H_2O$ langsam zugetropft und die entstehende orange Suspension während 16 Std. weiter gerührt. Der gelbe Niederschlag wird filtriert und mit je 20 ml Methanol (50%) und 20 ml $Et_2O$ gewaschen. (Die Aufarbeitung des erhaltenen Filtrats **I** wird in Beispiel A9 beschrieben.) Eine zweifache Umkristallisation aus Aceton ergibt 1,4 g [Pd((R)-(+)-$C_6H_4CHCH_3$-$N(CH_3)_2$(**H**)]] (Ausbeute: 61%). $^{31}P$-NMR ($CDCl_3$): 57,0 (d, J=43,8), 4,7 (d, J=43,8). 1,2 g [Pd((R)-(+)-$C_6H_4CHCH_3N(CH_3)_2$(**H**)]] (1,19 mmol) werden in einem Gemsich von 9 ml Aceton und 0,8 ml Salzsäure (10 N) gelöst und während 10 min bei Rückflusstemperatur gerührt. Anschliessend werden 20 ml Wasser bei Raumtemperatur hinzugefügt. Die entstehende orange Suspension wird konzentriert und filtriert. Der orangerote Festkörper wird mit Wasser nachgewaschen und am Hockvakuum getrocknet. Zu einer Suspension dieses Festkörpers in 20 ml Methanol werden 3,0 g KCN (46,1 mmol) addiert. Nach der Zugabe von 10 ml Wasser und 15 ml Dichloromethan bilden sich zwei Phasen. Die organische Phase wird über $MgSO_4$ getrocknet und eingeengt. Eine chromatographische Reinigung des gebildeten orangen Puders (bas. Alox, Hexan/Ethylacetat=9:1) ergibt quantitativ den freien Liganden **H**. Nach einer Umkristallisation aus Ethanol erhält man den diastereomerenreinen Liganden mit einer Ausbeute von 0,589 g (81,4%). $^{31}P$-NMR ($CDCl_3$): -6,1 (d, J=16), -25,5 (d, J=16).

Beispiel A9: {($S_p$)-(R)-1-[(S)-2-(Diphenylphosphino)ferrocenyl]}ethyl-(2-methoxyphenyl)phenylphosphin (**J**).

Es wird analog Beispiel A8 verfahren. Das in Beispiel A8 erhaltene Filtrat **I** wird mit einer Lösung von 0,5 g $NH_4PF_6$ (3,1 mmol) in 6 ml Wasser umgesetzt. Der braun-rote Niederschlag wird filtriert und mit 20 ml wässrigem Methanol (50%) und 20 ml $Et_2O$ nachgewaschen. Nach einer zweimaligen Umkristallisation aus Aceton/$Et_2O$ erhält man 1,2 g rote Kristalle (Ausbeute: 52%). $^{31}P$-NMR ($CDCl_3$): 30,6 (d, J=43,5), 36,2 (d, J=43,5). 1,2 g [Pd((R)-(+)-$C_6C_4CHCH_3N(CH_3)_2$(**J**)]] (1,19 mmol) werden analog zu Beispiel A8 mit KCN umgesetzt und man erhält 0,617 g (Ausbeute: 85%) diastereomerenreines **J**. $^{31}P$-NMR ($CDCl_3$): -7,9 (d, J=14), -25,1 (d, J=14).

Beispiel A10: {(R)-1-[(S)-2-(Diphenylphosphino)ferrocenyl]}ethyl-di-(4-trifluoromethylphenyl)phosphin (**K**).

Es wird analog Beispiel A1 verfahren, aber 1,0 g (R)-(S)-PPFOAc (2,2 mmol) und 0,82 g Di-(4-trifluoromethylphenyl)phosphin (2,55 mmol) verwendet. Die Ausbeute beträgt 1,20 g **K** (76%) als orange kristalline Substanz. $^{31}P$-NMR ($CDCl_3$): 6,6 (d, J=24), - 26,4 (d,J=24).

B) Anwendungsbeispiele

Beispiel B1: Herstellung von N-Acetylalaninmethylester.

In einem 200 ml Glasreaktor wird unter Argonatmosphäre mittels einer Stahlkapillare eine Katalysatorlösung (unter Argon hergestellt) transferiert, die aus 12,9 mg (0,034 mmol) [Rh (Norbornadien)$_2$]$BF_4$, 19,0 mg (0,037 mmol) A und 5 ml Methanol besteht. Anschliessend wir analog eine Lösung, bestehend aus 750 mg (3,42 mmol) Z-Methyl-2-acetamidocinnamat (Substrat) und 5 ml Methanol zugegeben. Das Molverhältnis Substrat/Katalysator beträgt 100. Dann werden in drei Spülzyklen 0,1 MPa Wasserstoff aufgepresst und der Wasserstoffdruck auf 0,108 MPa eingestellt. Die Reaktionsmischung wird bei 25°C 30 Minuten lang gerührt und darauf in einen Kolben übergeführt und das Lösungsmittel am Rotationsverdampfer entfernt. Bei einem chemischen Umsatz von 100 % erhält man N-Acetylalaninmethylester mit einem Enantiomerenüberschuss (ee) von 94 % (R).

Beispiel B2: Herstellung von N-Acetylalaninmethylester

Es wird analog Beispiel B1 verfahren und die Reaktionsbedingungen wie folgt abgewandelt: 24.4 mg (0,037 mmol) B; Reaktionszeit 1 Stunde; 50 bar Wasserstoffdruck. Der Umsatz ist 100%, ee: 83% (R).

Beispiel B3: Herstellung von N-Acetylalaninmethylester

Es wird analog Beispiel B1 verfahren und die Reaktionsbedingungen wie folgt abgewandelt: [Rh(Norbornadien)$_2$]$BF_4$ 8,3 mg (0,022 mmol); 15 mg (0,025 mmol) C (Verhältnis der beiden Stereoisomeren 1); Z-Methyl-2-acetamidocinnamat 0,49 g (2,22 mmol); Reaktionszeit 2 Stunden. Der Umsatz ist 100%, ee: 79% (**R**).

Beispiel B4: Herstellung von Dimethyl-2-methylsuccinat

Es wird analog Beispiel B1 verfahren und die Reaktionsbedingungen wie folgt abgewandelt: [Rh(Norbornadien)$_2$]$BF_4$ 11,8 mg (0,032 mmol); 20,7 mg (0,035 mmol) A; Dimethylitaconat (Substrat) 0,5 g (3.2 mmol); Reaktionszeit 30 Minuten. Der Umsatz ist 100%, ee >95%.

Beispiel B5: Herstellung von N-Acetylalaninmethylester

Es wird analog Beispiel B1 verfahren und die Reaktionsbedingungen wie folgt abgewandelt: [Rh((Norbornadien)Cl]$_2$: 3,9 mg (0,0085 mmol); **E**: 10,3 mg (0,019 mmol); Lösungsmittel: 11 ml Toluol/Methanol (10:1); Wasserstoff: 15 bar; Temperatur: 0 °C.; Reaktionsdauer: 20 Stunden. Der Umsatz ist 100%, ee: 73,6% (R).

Beispiel B6: Herstellung von N-(2',6'-Dimethylphenyl)-1-methoxymethyl-ethylamin.

Es wird analog Beispiel B1 verfahren und die Reaktionsbedingungen wie folgt abgewandelt: Es wird eine Katalysatorlösung bestehend aus 5,3 mg $[Ir((1,5\text{-Cyclooctadien})Cl]_2$ (0,0079 mmol),10,2 mg A (0,017 mmol) und 11,9 mg Tetrabutylammoniumiodid (0,032 mmol) in 5 ml Tetrahydrofuran/Dichloromethan (1:1) hergestellt. Ferner wird eine Substratlösung bestehend aus 1,5 g N-(2',6'-Dimethylphenyl)-1-methoxymethyl-ethylidenamin (7,8 mmol) in 5 ml Tetrahydrofuran verwendet. Es werden 40 bar Wasserstoff aufgepresst; die Reaktionstemperatur beträgt 30 °C.. Nach 91 Stunden ist der Umsatz 90%, der ee beträgt 59% (R).

Beispiel B7: Herstellung von N-(2',6'-Dimethylphenyl)-1-methoxymethyl-ethylamin.

Es wird analog Beispiel B6 verfahren und die Reaktionsbedingungen wie folgt abgewandelt: Katalysatorlösung bestehend aus 5,3 mg $[Ir((1,5\text{-Cyclooctadien})Cl]_2$ (0,0079 mmol) und 11,1 mg **G** (0,017 mmol); Reaktionszeit: 20 Stunden. Der Umsatz ist 100%, ee: 56,6% (S).

Beispiel B8: Herstellung von Methyl-2-hydroxy-2-phenylacetat

Es wird analog Beispiel B1 verfahren und die Reaktionsbedindungen wie folgt abgewandelt: $[Rh((\text{Norbornadien})Cl]_2$: 3,9 mg (0,0082 mmol); **E**: 9,7 mg (0,018 mmol); Methylphenylglyoxylat (Substrat): 0,268 mg (1,64 mmol); Lösungsmittel: 10 ml Toluol; Wasserstoff: 40 bar; Temperatur: 70 °C.; Reaktiondauer 20 Stunden. Der Umsatz ist 100%, ee: 41 %.

Beispiel B9: Herstellung von N-Acetylalanin

Es wird analog Beispiel B1 verfahren und die Reaktionbedingungen wie folgt abgewandelt: Acetamidozimtsäure (Substrat): 0,83 g (3,43 mmol). Der Umsatz ist 100%, ee: 81% (R).

Beispiel B10: Herstellung von N-Acetylalaninmethylester

Es wird analog Beispiel B1 verfahren und die Reaktionsbedingungen wie folgt abgewandelt: **H**: 18,3 mg (0,037 mmol). Der Umsatz beträgt 100%, ee: 79% (R).

Beispiel B11: Herstellung von Ethyl-4-phenyl-2-hydroxybutyrat

Es wird analog Beispiel B1 verfahren und die Reaktionsbedingungen wie folgt abgewandelt: Ethyl-4-phenyl-2-oxobutyrat (Substrat): 0,352 g (1,7 mmol); Lösungsmittel: 10 ml Toluol/Methanol (1:1); Wasserstoff: 50 bar; Temperatur: 50 °C.; Reaktionsdauer 19 Stunden. Der Umsatz ist 100%, ee: 44% (R).

## Patentansprüche

1. Verbindungen der Formel I,

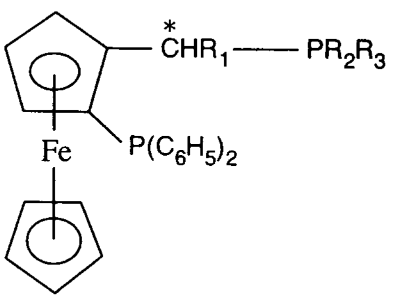

(I),

worin $R_1$ $C_1$-$C_8$-Alkyl, Phenyl oder mit 1 bis 3 $C_1$-$C_4$-Alkyl oder $C_1$-$C_4$-Alkoxy substituiertes Phenyl bedeuten; $R_2$ und $R_3$ gleich sind und $C_1$-$C_{12}$-Alkyl, $C_5$-$C_{12}$-Cycloalkyl, 4-Trifluoromethylphenyl im Falle $R_1$ gleich Methyl, mit $C_1$-$C_4$-Alkyl oder $C_1$-$C_4$-Alkoxy substituiertes $C_5$-$C_{12}$-Cycloalkyl oder mit ein bis drei $C_1$-$C_4$-Alkyl, $C_1$-$C_4$-Alkoxy, -$SiR_4R_5R_6$, Halogen, -$SO_3M$, -$CO_2M$, -$PO_3M$, -$NR_7R_8$ oder -$[^+NR_7R_8R_9]X^-$ substituiertes Phenyl darstellen; oder $R_2$

und $R_3$ verschieden sind und $C_1$-$C_{12}$-Alkyl, $C_5$-$C_{12}$-Cycloalkyl, mit $C_1$-$C_4$-Alkyl oder $C_1$-$C_4$-Alkoxy substituiertes $C_5$-$C_{12}$-Cycloalkyl, Phenyl oder mit ein bis drei $C_1$-$C_4$-Alkyl, $C_1$-$C_4$-Alkoxy, $-SiR_4R_5R_6$, Halogen, $-SO_3M$, $-CO_2M$, -$PO_3M$, $-NR_7R_8$ oder $-[^+NR_7R_8R_9]X^-$ substituiertes Phenyl bedeuten; oder die Gruppe $-PR_2R_3$ einen Rest der Formel II

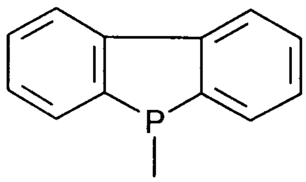

(II)

darstellt, und $R_4$, $R_5$ und $R_6$ unabhängig voneinander für $C_1$-$C_{12}$-Alkyl oder Phenyl stehen, $R_7$ und $R_8$ H, $C_1$-$C_{12}$-Alkyl, Phenyl oder $R_7$ und $R_8$ zusammen Tetramethylen, Pentamethylen oder 3-Oxa-1,5-Pentylen darstellen, $R_9$ H oder $C_1$-$C_4$-Alkyl bedeutet, M für H oder oder ein Alkalimetall steht, $X^-$ das Anion einer einbasischen Säure ist, und * ein stereogenes C-Atom kennzeichnet, in Form Ihrer Racemate und Diastereomeren oder Gemischen von Diastereomeren, ausgenommen Verbindungen der Formel I, worin $R_1$ $CH_3$ ist und die Gruppe $-PR_2R_3$ einen Rest der Formel II oder -P(cyclohexyl)$_2$ bedeutet.

2. Verbindungen der Formel I gemäss Anspruch 1, dadurch gekennzeichnet, dass $R_1$ als Alkyl linear ist.

3. Verbindungen der Formel I gemäss Anspruch 2, dadurch gekennzeichnet, dass $R_1$ $C_1$-$C_4$-Alkyl darstellt.

4. Verbindungen der Formel I gemäss Anspruch 3, dadurch gekennzeichnet, dass $R_1$ für Methyl oder Ethyl steht.

5. Verbindungen der Formel I gemäss Anspruch 1, dadurch gekennzeichnet, dass $R_1$ Phenyl oder mit 1 oder 2 $C_1$-$C_4$-Alkyl oder $C_1$-$C_4$-Alkoxy substituiertes Phenyl darstellt.

6. Verbindungen der Formel I gemäss Anspruch 1, dadurch gekennzeichnet, dass $R_2$ und $R_3$ als Alkyl linear oder verzweigt sind.

7. Verbindungen der Formel I gemäss Anspruch 6, dadurch gekennzeichnet, dass $R_2$ und $R_3$ $C_1$-$C_8$-Alkyl darstellen.

8. Verbindungen der Formel I gemäss Anspruch 6, dadurch gekennzeichnet, dass $R_2$ und $R_3$ gleich sind und für i-Propyl oder t-Butyl stehen.

9. Verbindungen der Formel I gemäss Anspruch 1, dadurch gekennzeichnet, dass $R_2$ und $R_3$ als Cycloalkyl 5 bis 8 Ring-C-Atome enthält.

10. Verbindungen der Formel I gemäss Anspruch 9, dadurch gekennzeichnet, dass $R_2$ und $R_3$ als Cycloalkyl Cyclopentyl oder Cyclohexyl darstellt.

11. Verbindungen der Formel I gemäss Anspruch 1, dadurch gekennzeichnet, dass $R_2$ und $R_3$ als substituiertes Phenyl 2-Methyl-, 3-Methyl-, 4-Methyl-, 2- oder 4-Ethyl-, 2- oder 4-i-Propyl-, 2- oder 4-t-Butyl-, 2-Methoxy-, 3-Methoxy-, 4-Methoxy-, 2- oder 4-Ethoxy-, 4-Trimethylsilyl-, 2- oder 4-Fluor-, 2,4-Difluor-, 2- oder 4-Chlor-, 2,4-Dichlor-, 2,4-Dimethyl-, 3,5-Dimethyl-, 2-Methoxy-4-methyl-, 3,5-Dimethyl-4-methoxy-, 3,5-Dimethyl-4-(dimethylamino)-, 2- oder 4-Amino-, 2- oder 4-Methylamino-, 2- oder 4-(Dimethylamnio)-, 2-oder 4-$SO_3H$-, 2- oder 4-$SO_3Na$-, 2- oder 4-$[^+NH_3Cl^-]$-, 2,4,6-Trimethylphen-1-yl oder 3,4,5-Trimethylphen-1-yl darstellen.

12. Verbindungen der Formel I gemäss Anspruch 1, dadurch gekennzeichnet, dass $R_2$ und $R_3$ gleich sind und Cyclohexyl, 2- oder 4-Methylphen-1-yl, 2- oder 4-Methoxyphen-1-yl, 2-oder 4-(Dimethylamino)phen-1-yl, 3,5-Dimethyl-4-(dimethylamino)phen-1-yl und 3,5-Dimethyl-4-methoxyphen-1-yl bedeuten.

13. Verbindungen der Formel I gemäss Anspruch 1, dadurch gekennzeichnet, dass $R_2$ und $R_3$ verschieden sind und $R_2$ Phenyl und $R_3$ Cyclohexyl, 2- oder 4-Methylphen-1-yl, 2- oder 4-Methoxyphen-1-yl, 4-(Dimethylamino)phen-1-yl, 3,5-Dimethyl-4-(dimethylamino)phen-1-yl, 3,5-Dimethyl-4-methoxyphen-1-yl oder 4-t-Butyl-phen-1-yl bedeuten.

**14.** Verbindungen der Formel I gemäss Anspruch 1, dadurch gekennzeichnet, dass $R_2$ und $R_3$ gleiche Reste darstellen und Cyclohexyl bedeuten.

**15.** Verbindungen der Formel I gemäss Anspruch 1, dadurch gekennzeichnet, dass $R_2$ und $R_3$ gleiche Reste darstellen und tert.-Butyl oder o-Anisyl bedeuten.

**16.** Verbindungen der Formel I gemäss Anspruch 1, dadurch gekennzeichnet, dass $R_2$ für Phenyl und $R_3$ für o-Anisyl stehen.

**17.** Verbindungen der Formel I gemäss Anspruch 1, dadurch gekennzeichnet, dass $R_1$ für Methyl und $R_2$ und $R_3$ für 4-Trifluoromethylphenyl stehen.

**18.** Verfahren zur Herstellung von Verbindungen der Formel I gemäss Anspruch 1, dadurch gekennzeichnet, dass man eine Verbindung der Formel III

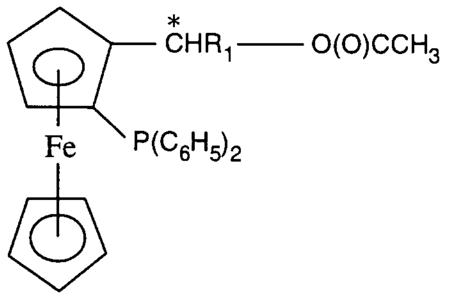

(III)

in Gegenwart eines inerten Lösungsmittels bei erhöhter Temperatur mit einem Phosphin der Formel IV

$$HPR_2R_3 \qquad \text{(IV)}$$

umsetzt, wobei $R_1$, $R_2$ und $R_3$ die in Anspruch 1 angegebenen Bedeutungen haben.

**19.** Komplexe der Formeln V und VI,

$$[X_1M_1YZ] \qquad \text{(V)},$$

$$[X_1M_1Y]^+A_1^- \qquad \text{(VI)},$$

worin $X_1$ für zwei $C_2$-$C_{12}$-Olefine oder ein $C_5$-$C_{12}$-Dien steht, Z für Cl, Br oder I steht, $A_1^-$ das Anion einer Sauerstoffsäure oder Komplexsäure bedeutet, $M_1$ für Rh oder Ir steht und Y eine Verbindung der Formel I

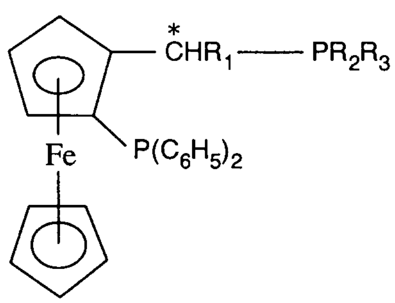

(I),

worin $R_1$ $C_1$-$C_8$-Alkyl, Phenyl oder mit 1 bis 3 $C_1$-$C_4$-Alkyl oder $C_1$-$C_4$-Alkoxy substituiertes Phenyl bedeuten; $R_2$ und $R_3$ gleich sind und $C_1$-$C_{12}$-Alkyl, $C_5$-$C_{12}$-Cycloalkyl, 4-Trifluoromethylphenyl im Falle $R_1$ gleich Methyl, mit $C_1$-$C_4$-Alkyl oder $C_1$-$C_4$-Alkoxy substituiertes $C_5$-$C_{12}$-Cycloalkyl oder mit ein bis drei $C_1$-$C_4$-Alkyl, $C_1$-$C_4$-Alkoxy, -

$SiR_4R_5R_6$, Halogen, $-SO_3M$, $-CO_2M$, $-PO_3M$, $-NR_7R_8$ oder $-[^+NR_7R_8R_9]X^-$ substituiertes Phenyl darstellen; oder $R_2$ und $R_3$ verschieden sind und $C_1$-$C_{12}$-Alkyl, $C_5$-$C_{12}$-Cycloalkyl, mit $C_1$-$C_4$-Alkyl oder $C_1$-$C_4$-Alkoxy substituiertes $C_5$-$C_{12}$-Cycloalkyl, Phenyl oder mit ein bis drei $C_1$-$C_4$-Alkyl, $C_1$-$C_4$-Alkoxy, $-SiR_4R_5R_6$, Halogen, $-SO_3M$, $-CO_2M$, $-PO_3M$, $-NR_7R_8$ oder $-[^+NR_7R_8R_9]X^-$ substituiertes Phenyl bedeuten; oder die Gruppe $-PR_2R_3$ einen Rest der Formel II

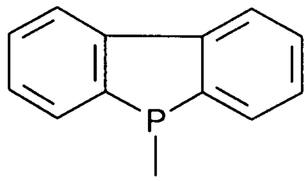

(II)

darstellt, und $R_4$, $R_5$ und $R_6$ unabhängig voneinander für $C_1$-$C_{12}$-Alkyl oder Phenyl stehen, $R_7$ und $R_8$ H, $C_1$-$C_{12}$-Alkyl, Phenyl oder $R_7$ und $R_8$ zusammen Tetramethylen, Pentamethylen oder 3-Oxa-1,5-Pentylen darstellen, $R_9$ H oder $C_1$-$C_4$-Alkyl bedeutet, M für H oder oder ein Alkalimetall steht, $X^-$ das Anion einer einbasischen Säure ist, und * ein stereogenes C-Atom kennzeichnet, in Form Ihrer Racemate und Diastereomeren oder Gemischen von Diastereomeren darstellt.

**20.** Komplexe gemäss Anspruch 19, dadurch gekennzeichnet, dass es sich um solche der Formel VI handelt.

**21.** Komplexe gemäss Anspruch 19, dadurch gekennzeichnet, dass $X_1$ zwei Ethylen, 1,5-Hexadien, 1,5-Cyclooctadien oder Norbornadien darstellen.

**22.** Komplexe gemäss Anspruch 19, dadurch gekennzeichnet, dass Z für Cl oder Br steht.

**23.** Komplexe gemäss Anspruch 19, dadurch gekennzeichnet, dass Z für $ClO_4^-$, $FSO_3^-$, $CH_3SO_3^-$, $CF_3SO_3^-$, $BF_4^-$, $PF_6^-$, $SbCl_6^-$, $AsF_6^-$ und $SbF_6^-$ steht.

**24.** Komplexe gemäss Anspruch 19, dadurch gekennzeichnet, dass $R_1$ als Alkyl linear ist.

**25.** Komplexe gemäss Anspruch 24, dadurch gekennzeichnet, dass $R_1$ $C_1$-$C_4$-Alkyl darstellt.

**26.** Komplexe gemäss Anspruch 25, dadurch gekennzeichnet, dass $R_1$ für Methyl oder Ethyl steht.

**27.** Komplexe gemäss Anspruch 19, dadurch gekennzeichnet, dass $R_1$ Phenyl oder mit 1 oder 2 $C_1$-$C_4$-Alkyl oder $C_1$-$C_4$-Alkoxy substituiertes Phenyl darstellt.

**28.** Komplexe gemäss Anspruch 19, dadurch gekennzeichnet, dass $R_2$ und $R_3$ als Alkyl linear oder verzweigt sind.

**29.** Komplexe gemäss Anspruch 28, dadurch gekennzeichnet, dass $R_2$ und $R_3$ $C_1$-$C_8$-Alkyl darstellen.

**30.** Komplexe gemäss Anspruch 28, dadurch gekennzeichnet, dass $R_2$ und $R_3$ gleich sind und für i-Propyl oder t-Butyl stehen.

**31.** Komplexe gemäss Anspruch 19, dadurch gekennzeichnet, dass $R_2$ und $R_3$ als Cycloalkyl 5 bis 8 Ring-C-Atome enthält.

**32.** Komplexe gemäss Anspruch 31, dadurch gekennzeichnet, dass $R_2$ und $R_3$ als Cycloalkyl Cyclopentyl oder Cyclohexyl darstellt.

**33.** Komplexe gemäss Anspruch 19, dadurch gekennzeichnet, dass $R_2$ und $R_3$ als substituiertes Phenyl 2-Methyl-, 3-Methyl-, 4-Methyl-, 2- oder 4-Ethyl-, 2- oder 4-i-Propyl-, 2- oder 4-t-Butyl-, 2-Methoxy-, 3-Methoxy-, 4-Methoxy-, 2- oder 4-Ethoxy-, 4-Trimethylsilyl-, 2- oder 4-Fluor-, 2,4-Difluor-, 2- oder 4-Chlor-, 2,4-Dichlor-, 2,4-Dimethyl-, 3,5-Dimethyl-, 2-Methoxy-4-methyl-, 3,5-Dimethyl-4-methoxy-, 3,5-Dimethyl-4-(dimethylamino)-, 2- oder 4-Amino-, 2- oder 4-Methylamino-, 2- oder 4-(Dimethylamnio)-, 2- oder 4-$SO_3H$-, 2- oder 4-$SO_3Na$-, 2- oder 4-$[^+NH_3Cl^-]$-, 2,4,6-

Trimethylphen-1-yl oder 3,4,5-Trimethylphen-1-yl darstellen.

**34.** Komplexe gemäss Anspruch 19, dadurch gekennzeichnet, dass $R_2$ und $R_3$ gleich sind und Cyclohexyl, 2- oder 4-Methylphen-1-yl, 2- oder 4-Methoxyphen-1-yl, 2- oder 4-(Dimethylamino)phen-1-yl, 3,5-Dimethyl-4-(dimethylamino)phen-1-yl und 3,5-Dimethyl-4-methoxyphen-1-yl bedeuten.

**35.** Komplexe gemäss Anspruch 19, dadurch gekennzeichnet, dass $R_2$ und $R_3$ verschieden sind und $R_2$ Phenyl und $R_3$ Cyclohexyl, 2- oder 4-Methylphen-1-yl, 2-oder 4-Methoxyphen-1-yl, 4-(Dimethylamino)phen-1-yl, 3,5-Dimethyl-4-(dimethylamino)phen-1-yl, 3,5-Dimethyl-4-methoxyphen-1-yl oder 4-t-Butyl-phen-1-yl bedeuten.

**36.** Komplexe gemäss Anspruch 19, dadurch gekennzeichnet, dass $R_2$ und $R_3$ gleiche Reste darstellen und Cyclohexyl bedeuten.

**37.** Komplexe gemäss Anspruch 19, dadurch gekennzeichnet, dass $R_2$ und $R_3$ gleiche Reste darstellen und tert.-Butyl oder o-Anisyl bedeuten.

**38.** Komplexe gemäss Anspruch 19, dadurch gekennzeichnet, dass $R_2$ für Phenyl und $R_3$ für o-Anisyl stehen.

**39.** Komplexe gemäss Anspruch 19, dadurch gekennzeichnet, dass $R_1$ Methyl und $R_2$ und $R_3$ je Cyclohexyl bedeuten.

**40.** Verwendung der Komplexe der Formeln V und VI gemäss Anspruch 19 als homogene Katalysatoren zur asymmetrischen Hydrierung von prochiralen Verbindungen mit Kohlenstoff-oder Kohlenstoff-/Heteroatomdoppelbindungen.

**41.** Verwendung gemäss Anspruch 40 zur Hydrierung von unsymmetrischen Kohlenstoffdoppelbindungen, Ketiminen und Ketonen.

**42.** Verwendung gemäss Anspruch 40 zur Hydrierung von unsymmetrischen Kohlenstoffdoppelbindungen mit den Rhodium-Komplexen der Formeln V und VI.

**43.** Verfahren zur asymmetrischen Hydrierung von Verbindungen mit Kohlenstoff-oder Kohlenstoff-/Heteroatomdoppelbindungen unter homogenen Reaktionsbedingungen, dadurch gekennzeichnet, dass man die Verbindungen bei einer Temperatur von - 20 bis 80 °C und einem Wasserstoffdruck von $10^4$ bis $10^7$ Pa in Gegenwart katalytischer Mengen eines Komplexes der Formeln V oder VI gemäss Anspruch 19 hydriert.

**44.** Verfahren gemäss Anspruch 43, dadurch gekennzeichnet, dass der Wasserstoffdruck $10^5$ bis $6.10^6$ Pa beträgt.

**45.** Verfahren gemäss Anspruch 43, dadurch gekennzeichnet, dass die Temperatur - 10 bis 50 °C beträgt.

**46.** Verfahren gemäss Anspruch 43, dadurch gekennzeichnet, dass die Menge des Katalysators so gewählt wird, dass das Molverhältnis der zu hydrierenden Verbindung (Substrat) zu dem Komplex der Formeln V oder VI 10000 bis 20 beträgt.

**47.** Verfahren gemäss Anspruch 43, dadurch gekennzeichnet, dass die Reaktion in Gegenwart eines Lösungsmittels durchgeführt wird.

**48.** Verfahren gemäss Anspruch 43, dadurch gekennzeichnet, dass das Lösungsmittel Methanol oder eine Mischung von Methanol mit Benzol oder Toluol ist.

## Claims

**1.** Compounds of formula I,

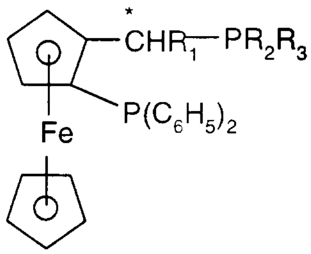

(I),

wherein $R_1$ is $C_1$-$C_8$-alkyl, phenyl or phenyl which is substituted by 1 to 3 $C_1$-$C_4$-alkyl or $C_1$-$C_4$-alkoxy; $R_2$ and $R_3$ are identical and are $C_1$-$C_{12}$-alkyl, $C_5$-$C_{12}$-cycloalkyl, 4-trifluoromethyl in the case where $R_1$ is identical to methyl, or $C_1$-$C_4$-alkyl- or $C_1$-$C_4$-alkoxy-substituted $C_5$-$C_{12}$-cycloalkyl, or phenyl which is substituted by one to three $C_1$-$C_4$-alkyl, $C_1$-$C_4$-alkoxy, $-SiR_4R_5R_6$, halogen, $-SO_3M$, $-CO_2M$, $-PO_3M$, $-NR_7R_8$ or $-[^{\oplus}NR_7R_8R_9]X^{\theta}$; or $R_2$ and $R_3$ are different and are $C_1$-$C_{12}$-alkyl, $C_5$-$C_{12}$-cycloalkyl, $C_1$-$C_4$-alkyl- or $C_1$-$C_4$-alkoxy-substituted $C_5$-$C_{12}$-cycloalkyl, phenyl or phenyl which is substituted by one to three $C_1$-$C_4$-alkyl, $C_1$-$C_4$-alkoxy, $-SiR_4R_5R_6$, halogen, $-SO_3M$, $-CO_2M$, $-PO_3M$, $-NR_7R_8$ or $-[^{\oplus}NR_7R_8R_9]X^{\theta}$; or the group $-PR_2R_3$ is a radical of formula II

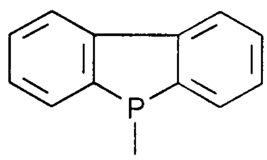

(II)

and $R_4$, $R_5$ and $R_6$, independently of one another, are $C_1$-$C_{12}$-alkyl or phenyl, $R_7$ and $R_8$ are H, $C_1$-$C_{12}$-alkyl, phenyl or $R_7$ and $R_8$ together are tetramethylene, pentamethylene or 3-oxa-1,5-pentylene, $R_9$ is H or $C_1$-$C_4$-alkyl, M is H or an alkali metal, $X^{\theta}$ is the anion of a monobasic acid, and * is a stereogenic carbon atom, in the form of their racemates and diastereoisomers or mixtures of diastereoisomers, with the exception of compounds of formula I, wherein $R_1$ is $CH_3$ and the group $-PR_2R_3$ signifies a radical of formula II or P(cyclohexyl)$_2$.

2. Compounds of formula I according to claim 1, characterised in that $R_1$ as alkyl is linear.

3. Compounds of formula I according to claim 2, characterised in that $R_1$ is $C_1$-$C_4$-alkyl.

4. Compounds of formula I according to claim 3, characterised in that $R_1$ is methyl or ethyl.

5. Compounds of formula I according to claim 1, characterised in that $R_1$ is phenyl or phenyl which is substituted by 1 or 2 $C_1$-$C_4$-alkyl or $C_1$-$C_4$-alkoxy groups.

6. Compounds of formula I according to claim 1, characterised in that $R_2$ and $R_3$ as alkyl are linear or branched.

7. Compounds of formula I according to claim 6, characterised in that $R_2$ and $R_3$ are $C_1$-$C_8$-alkyl.

8. Compounds of formula I according to claim 6, characterised in that $R_2$ and $R_3$ are identical and are isopropyl or tert.-butyl.

9. Compounds of formula I according to claim 1, characterised in that $R_2$ and $R_3$ as cycloalkyl contain 5 to 8 ring carbon atoms.

10. Compounds of formula I according to claim 9, characterised in that $R_2$ and $R_3$ as cycloalkyl are cyclopentyl or cyclohexyl.

11. Compounds of formula I according to claim 1, characterised in that $R_2$ and $R_3$ as substituted phenyl are 2-methyl-, 3-methyl-, 4-methyl-, 2- or 4-ethyl-, 2- or 4-i-propyl-, 2- or 4-t-butyl-, 2-methoxy-, 3-methoxy-, 4-methoxy-, 2- or 4-ethoxy-, 4-trimethylsilyl-, 2- or 4-fluoro-, 2,4-difluoro-, 2- or 4-chloro-, 2,4-dichloro-, 2,4-dimethyl-, 3,5-dimethyl-, 2-methoxy-4-methyl-, 3,5-dimethyl-4-methoxy-, 3,5-dimethyl-4-(dimethylamino)-, 2- or 4-amino-, 2- or 4-methylamino-, 2- or 4-(dimethylamino)-, 2- or 4-$SO_3H$-, 2- or 4-$SO_3Na$-, 2- or 4-[$^{\oplus}NH_3Cl^{\theta}$]-, 2,4,6-trimethylphen-1-yl or 3,4,5-trimethylphen-1-yl.

**12.** Compounds of formula I according to claim 1, characterised in that $R_2$ and $R_3$ are identical and are cyclohexyl, 2- or 4-methylphen-1-yl, 2- or 4-methoxyphen-1-yl, 2- or 4-(dimethylamino)phen-1-yl, 3,5-dimethyl-4-(dimethylamino)phen-1-yl and 3,5-dimethyl-4-methoxyphen-1-yl.

**13.** Compounds of formula I according to claim 1, characterised in that $R_2$ and $R_3$ are different and $R_2$ is phenyl and $R_3$ is cyclohexyl, 2- or 4-methylphen-1-yl, 2- or 4-methoxyphen-1-yl, 4-(dimethylamino)-phen-1-yl, 3,5-dimethyl-4-(dimethylamino)phen-1-yl, 3,5-dimethyl-4-methoxyphen-1-yl or 4-t-butyl-phen-1-yl.

**14.** Compounds of formula I according to claim 1, characterised in that $R_2$ and $R_3$ are identical substituents and are cyclohexyl.

**15.** Compounds of formula I according to claim 1, characterised in that $R_2$ and $R_3$ are identical substituents and are tert.-butyl or o-anisyl.

**16.** Compounds of formula I according to claim 1, characterised in that $R_2$ is phenyl and $R_3$ is o-anisyl.

**17.** Compounds of formula I according to claim 1, characterised in that $R_1$ is methyl and $R_2$ and $R_3$ are 4-trifluoromethylphenyl.

**18.** Process for the preparation of compounds of formula I according to claim 1, characterised in that a compound of formula III

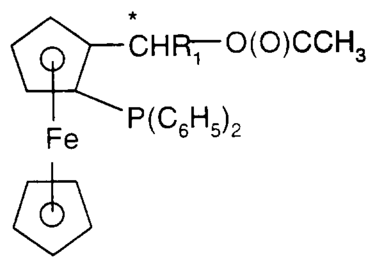

(III)

is reacted in the presence of an inert solvent at an elevated temperature, with a phosphine of formula IV

$$HPR_2R_3 \quad (IV)$$

whereby $R_1$, $R_2$ and $R_3$ have the significances given in claim 1.

**19.** Complexes of formulae V and VI,

$$[X_1M_1YZ] \quad (V),$$

$$[X_1M_1Y]^{\oplus}A_1^{\theta} \quad (VI),$$

wherein $X_1$ denotes two $C_2$-$C_{12}$-olefins or a $C_5$-$C_{12}$-diene, Z is Cl, Br or I, $A_1^{\theta}$ signifies the anion of an oxyacid or complex acid, $M_1$ is Rh or Ir and Y is a compound of formula I

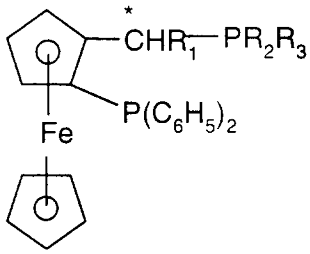

(I),

wherein $R_1$ is $C_1$-$C_8$-alkyl, phenyl or phenyl which is substituted by 1 to 3 $C_1$-$C_4$-alkyl or $C_1$-$C_4$-alkoxy; $R_2$ and $R_3$ are identical and are $C_1$-$C_{12}$-alkyl, $C_5$-$C_{12}$-cycloalkyl, 4-trifluoromethyl in the case where $R_1$ is identical to methyl, or $C_1$-$C_4$-alkyl- or $C_1$-$C_4$-alkoxy-substituted $C_5$-$C_{12}$-cycloalkyl, or phenyl which is substituted by one to three $C_1$-$C_4$-alkyl, $C_1$-$C_4$-alkoxy, $-SiR_4R_5R_6$, halogen, $-SO_3M$, $-CO_2M$, $-PO_3M$, $-NR_7R_8$ or $-[^{\oplus}NR_7R_8R_9]X^{\theta}$; or $R_2$ and $R_3$ are different and are $C_1$-$C_{12}$-alkyl, $C_5$-$C_{12}$-cycloalkyl, $C_1$-$C_4$-alkyl- or $C_1$-$C_4$-alkoxy-substituted $C_5$-$C_{12}$-cycloalkyl, phenyl or phenyl which is substituted by one to three $C_1$-$C_4$-alkyl, $C_1$-$C_4$-alkoxy, $-SiR_4R_5R_6$, halogen, $-SO_3M$, $-CO_2M$, $-PO_3M$, $-NR_7R_8$ or $-[^{\oplus}NR_7R_8R_9]X^{\theta}$; or the group $-PR_2R_3$ is a radical of formula II

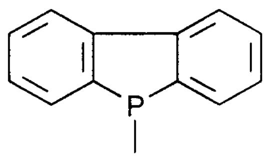

(II)

and $R_4$, $R_5$ and $R_6$, independently of one another, are $C_1$-$C_{12}$-alkyl or phenyl, $R_7$ and $R_8$ are H, $C_1$-$C_{12}$-alkyl, phenyl or $R_7$ and $R_8$ together are tetramethylene, pentamethylene or 3-oxa-1,5-pentylene, $R_9$ is H or $C_1$-$C_4$-alkyl, M is H or an alkali metal, $X^{\theta}$ is the anion of a monobasic acid, and * is a stereogenic carbon atom, in the form of their racemates and diastereoisomers or mixtures of diastereoisomers.

**20.** Complexes according to claim 19, characterised in that they are those of formula VI.

**21.** Complexes according to claim 19, characterised in that $X_1$ is two ethylene, 1,5-hexadiene, 1,5-cyclooctadiene or norbornadiene.

**22.** Complexes according to claim 19, characterised in that Z is Cl or Br.

**23.** Complexes according to claim 19, characterised in that Z is $ClO_4^{\theta}$, $FSO_3^{\theta}$, $CH_3SO_3^{\theta}$, $CF_3SO_3^{\theta}$, $BF_4^{\theta}$, $PF_6^{\theta}$, $SbCl_6^{\theta}$, $AsF_6^{\theta}$ and $SbF_6^{\theta}$.

**24.** Complexes according to claim 19, characterised in that $R_1$ as alkyl is linear.

**25.** Complexes according to claim 24, characterised in that $R_1$ is $C_1$-$C_4$-alkyl.

**26.** Complexes according to claim 25, characterised in that $R_1$ is methyl or ethyl.

**27.** Complexes according to claim 19, characterised in that $R_1$ is phenyl or phenyl which is substituted by 1 or 2 $C_1$-$C_4$-alkyl or $C_1$-$C_4$-alkoxy.

**28.** Complexes according to claim 19, characterised in that $R_2$ and $R_3$ as alkyl are linear or branched.

**29.** Complexes according to claim 28, characterised in that $R_2$ and $R_3$ are $C_1$-$C_8$-alkyl.

**30.** Complexes according to claim 28, characterised in that $R_2$ and $R_3$ are identical and are isopropyl or tert.-butyl.

**31.** Complexes according to claim 19, characterised in that $R_2$ and $R_3$ as cycloalkyl contain 5 to 8 ring carbon atoms.

**32.** Complexes according to claim 31, characterised in that $R_2$ and $R_3$ as cycloalkyl are cyclopentyl or cyclohexyl.

**33.** Complexes according to claim 19, characterised in that $R_2$ and $R_3$ as substituted phenyl are 2-methyl-, 3-methyl-, 4-methyl-, 2- or 4-ethyl-, 2- or 4-i-propyl-, 2- or 4-t-butyl-, 2-methoxy-, 3-methoxy-, 4-methoxy-, 2- or 4-ethoxy-, 4-trimethylsilyl-, 2- or 4-fluoro-, 2,4-difluoro-, 2- or 4-chloro-, 2,4-dichloro-, 2,4-dimethyl-, 3,5-dimethyl-, 2-methoxy-4-methyl-, 3,5-dimethyl-4-methoxy-, 3,5-dimethyl-4-(dimethylamino)-, 2- or 4-amino-, 2- or 4-methylamino-, 2- or 4-(dimethylamino)-, 2- or 4-$SO_3H$-, 2- or 4-$SO_3Na$-, 2- or 4-[$^{\oplus}NH_3Cl^{\ominus}$]-, 2,4,6-trimethylphen-1-yl or 3,4,5-trimethylphen-1-yl.

**34.** Complexes according to claim 19, characterised in that $R_2$ and $R_3$ are identical and are cyclohexyl, 2- or 4-methylphen-1-yl, 2- or 4-methoxyphen-1-yl, 2- or 4-(dimethylamino)phen-1-yl, 3,5-dimethyl-4-(dimethylamino)phen-1-yl and 3,5-dimethyl-4-methoxyphen-1-yl.

**35.** Complexes according to claim 19, characterised in that $R_2$ and $R_3$ are different and $R_2$ is phenyl and $R_3$ is cyclohexyl, 2- or 4-methylphen-1-yl, 2- or 4-methoxyphen-1-yl, 4-(dimethylamino)-phen-1-yl, 3,5-dimethyl-4-(dimethylamino)phen-1-yl, 3,5-dimethyl-4-methoxyphen-1-yl or 4-t-butyl-phen-1-yl.

**36.** Complexes according to claim 19, characterised in that $R_2$ and $R_3$ are identical substituents and are cyclohexyl.

**37.** Complexes according to claim 19, characterised in that $R_2$ and $R_3$ are identical substituents and are tert.-butyl or o-anisyl.

**38.** Complexes according to claim 19, characterised in that $R_2$ is phenyl and $R_3$ is o-anisyl.

**39.** Complexes according to claim 19, characterised in that $R_1$ is methyl and $R_2$ and $R_3$ are each cyclohexyl.

**40.** Use of the complexes of formulae V and VI according to claim 19 as homogeneous catalysts for the asymmetric hydrogenation of prochiral compounds containing carbon double bonds or carbon/hetero atom double bonds.

**41.** Use according to claim 40 for the hydrogenation of unsymmetric carbon double bonds, ketimines and ketones.

**42.** Use according to claim 40 for the hydrogenation of unsymmetric carbon double bonds with the rhodium complexes of formulae V and VI.

**43.** Process for the asymmetric hydrogenation of compounds containing carbon double bonds or carbon/hetero atom double bonds under homogeneous reaction conditions, characterised in that the compounds are hydrogenated at a temperature of - 20 to 80°C and under a hydrogen pressure of $10^4$ to $10^7$ Pa in the presence of catalytic amounts of a complex of formulae V or VI according to claim 19.

**44.** Process according to claim 43, characterised in that the hydrogen pressure is $10^5$ to $6 \cdot 10^6$ Pa.

**45.** Process according to claim 43, characterised in that the temperature is - 10 to 50°C.

**46.** Process according to claim 43, characterised in that the amount of catalyst is chosen such that the molar ratio of the compound to be hydrogenated (substrate) to the complex of formulae V or VI is 10,000 to 20.

**47.** Process according to claim 43, characterised in that the reaction is carried out in the presence of a solvent.

**48.** Process according to claim 43, characterised in that the solvent is methanol or a mixture of methanol with benzene or toluene.

## Revendications

**1.** Les composés de formule I

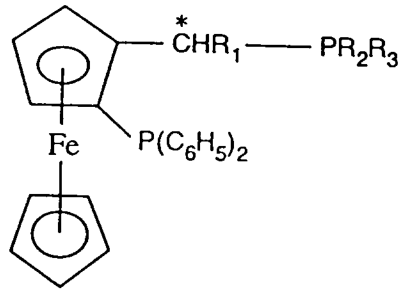

(I),

où $R_1$ signifie un groupe $C_1$-$C_8$-alkyle, phényle ou phényle substitué par 1 à 3 groupes $C_1$-$C_4$-alkyle ou $C_1$-$C_4$-alcoxy; $R_2$ et $R_3$ sont identiques et signifient un groupe $C_1$-$C_{12}$-alkyle, $C_5$-$C_{12}$-cycloalkyle, 4-trifluorométhylphényle lorsque $R_1$ représente un groupe méthyle, un groupe $C_5$-$C_{12}$-cycloalkyle substitué par un groupe $C_1$-$C_4$-alkyle ou $C_1$-$C_4$-alcoxy ou un groupe phényle substitué par 1 à 3 groupes $C_1$-$C_4$-alkyle, $C_1$-$C_4$-alcoxy, -Si-$R_4R_5R_6$, un halogène, -$SO_3M$, -$CO_2M$, -$PO_3M$, -$NR_7R_8$ ou -[$^+NR_7R_8R_9$]$X^-$; ou $R_2$ et $R_3$ sont différents et signifient un groupe $C_1$-$C_{12}$-alkyle, $C_5$-$C_{12}$-cycloalkyle, $C_5$-$C_{12}$-cycloalkyle substitué par un groupe $C_1$-$C_4$-alkyle ou $C_1$-$C_4$-alcoxy, phényle ou phényle substitué par 1 à 3 groupes $C_1$-$C_4$-alkyle, $C_1$-$C_4$-alcoxy, -Si-$R_4R_5R_6$, un halogène, -$SO_3M$, -$CO_2M$, -$PO_3M$, -$NR_7R_8$ ou -[$^+NR_7R_8R_9$]$X^-$; ou bien le groupe -$PR_2R_3$ signifie un reste de formule II

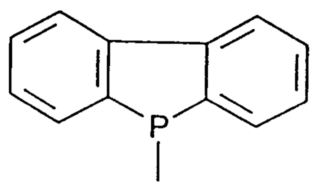

(II)

et $R_4$, $R_5$ et $R_6$ représentent indépendamment les uns des autres un groupe $C_1$-$C_{12}$-alkyle ou phényle, $R_7$ et $R_8$ représentent H, un groupe $C_1$-$C_{12}$-alkyle, phényle ou $R_7$ et $R_8$ signifient ensemble un groupe tétraméthylène, pentaméthylène ou 3-oxa-1,5-pentylène, $R_9$ signifie H ou un groupe $C_1$-$C_4$-alkyle, M signifie H ou un métal alcalin, $X^-$ signifie l'anion d'un acide monobasique et * caractérise un atome de carbone stéréogène, sous forme de leurs racémates et de leurs diastéréomères ou de mélanges de diastéréomères, à l'exception des composés de formule I où $R_1$ signifie $CH_3$ et le groupe -$PR_2R_3$ signifie un reste de formule II ou -P(cyclohexyle)$_2$.

2. Les composés de formule I selon la revendication 1, caractérisés en ce que $R_1$ en tant que groupe alkyle est linéaire.

3. Les composés de formule I selon la revendication 2, caractérisés en ce que $R_1$ représente un groupe $C_1$-$C_4$-alkyle.

4. Les composés de formule I selon la revendication 3, caractérisés en ce que $R_1$ signifie un groupe méthyle ou éthyle.

5. Les composés de formule I selon la revendication 1, caractérisés en ce que $R_1$ signifie un groupe phényle ou phényle substitué par 1 ou 2 groupes $C_1$-$C_4$-alkyle ou $C_1$-$C_4$-alcoxy.

6. Les composés de formule I selon la revendication 1, caractérisés en ce que $R_2$ et $R_3$ en tant que groupe alkyle sont linéaires ou ramifiés.

7. Les composés de formule I selon la revendication 6, caractérisés en ce que $R_2$ et $R_3$ représentent un groupe $C_1$-$C_8$-alkyle.

8. Les composés de formule I selon la revendication 6, caractérisés en ce que $R_2$ et $R_3$ sont identiques et représentent un groupe iso-propyle ou tert.-butyle.

9. Les composés de formule I selon la revendication 1, caractérisés en ce que $R_2$ et $R_3$ en tant que groupe cycloalkyle

contiennent de 5 à 8 atomes de carbone dans le cycle.

**10.** Les composés de formule I selon la revendication 9, caractérisés en ce que $R_2$ et $R_3$ en tant que groupe cycloalkyle représentent un groupe cyclopentyle ou cyclohexyle.

**11.** Les composés de formule I selon la revendication 1, caractérisés en ce que $R_2$ et $R_3$ en tant que groupe phényle substitué représentent un groupe 2-méthyl-, 3-méthyl-, 4-méthyl-, 2- ou 4-éthyl-, 2- ou 4-iso-propyl-, 2- ou 4-tert.-butyl-, 2-méthoxy-, 3-méthoxy-, 4-méthoxy-, 2- ou 4-éthoxy-, 4-triméthylsilyl-, 2-ou 4-fluoro-, 2,4-difluoro-, 2- ou 4-chloro-, 2,4-dichloro-, 2,4-diméthyl-, 3,5-diméthyl-, 2-méthoxy-4-méthyl-, 3,5-diméthyl-4-méthoxy-, 3,5-diméthyl-4-(diméthylamino)-, 2- ou 4-amino-, 2- ou 4-méthylamino, 2- ou 4-(diméthylamino), 2- ou 4-$SO_3H$-, 2- ou 4-$SO_3Na$-, 2- ou 4-[$^+NH_3Cl^-$]-, 2,4,6-triméthyl-phen-1-yle ou 3,4,5-triméthyl-phen-1-yle.

**12.** Les composés de formule I selon la revendication 1, caractérisés en ce que $R_2$ et $R_3$ sont identiques et signifient un groupe cyclohexyle, 2-ou 4-méthylphen-1-yle, 2- ou 4-méthoxyphen-1-yle, 2- ou 4-(diméthylamino)phen-1-yle, 3,5-diméthyl-4-(diméthylamino)phen-1-yle et 3,5-diméthyl-4-méthoxyphen-1-yle.

**13.** Les composés de formule I selon la revendication 1, caractérisés en ce que $R_2$ et $R_3$ sont différents et $R_2$ signifie un groupe phényle et $R_3$ signifie un groupe cyclohexyle, 2- ou 4-méthylphen-1-yle, 2- ou 4-méthoxyphen-1-yle, 4-(diméthylamino)phen-1-yle, 3,5-diméthyl-4-(diméthylamino)phen-1-yle, 3,5-diméthyl-4-méthoxyphen-1-yle ou 4-tert.-butyl-phen-1-yle.

**14.** Les composés de formule I selon la revendication 1, caractérisés en ce que $R_2$ et $R_3$ représentent des restes identiques et signifient un groupe cyclohexyle.

**15.** Tes composés de formule I selon la revendication 1, caractérisés en ce que $R_2$ et $R_3$ représentent des restes identiques et signifient un groupe tert.-butyle ou o-anisyle.

**16.** Tes composés de formule I selon la revendication 1, caractérisés en ce que $R_2$ signifie un groupe phényle et $R_3$ signifie un groupe o-anisyle.

**17.** Les composés de formule I selon la revendication 1, caractérisés en ce que $R_1$ signifie un groupe méthyle et $R_2$ et $R_3$ signifient un groupe 4-trifluorométhylphényle.

**18.** Un procédé de préparation des composés de formule I selon la revendication 1, caractérisé en ce qu'on fait réagir un composé de formule III

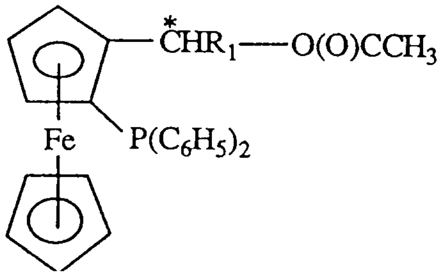

(III)

en présence d'un solvant inerte à une température élevée avec une phosphine de formule IV

$$HPR_2R_3 \qquad (IV)$$

$R_1$, $R_2$ et $R_3$ ayant les significations données à la revendication 1.

**19.** Tes complexes des formules V et VI,

$$[X_1M_1YZ] \qquad (V),$$

$$[X_1M_1Y]^+A_1^- \qquad (VI),$$

où $X_1$ signifie deux $C_2$-$C_{12}$-oléfines ou un $C_5$-$C_{12}$-diène, Z signifie Cl, Br ou I, $A_1^-$signifie l'anion d'un oxyacide ou d'un complexe acide, $M_1$ signifie Rh ou Ir et Y signifie un composé de formule I

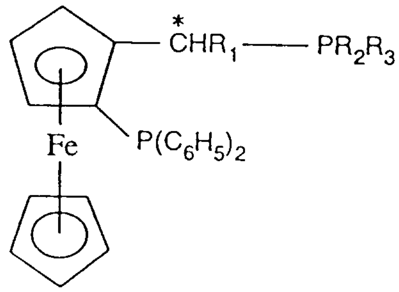

(I),

où $R_1$ signifie un groupe $C_1$-$C_8$-alkyle, phényle ou phényle substitué par 1 à 3 groupes $C_1$-$C_4$-alkyle ou $C_1$-$C_4$-alcoxy; $R_2$ et $R_3$ sont identiques et représentent un groupe $C_1$-$C_{12}$-alkyle, $C_5$-$C_{12}$-cycloalkyle, 4-trifluorométhylphényle lorsque $R_1$ signifie un groupe méthyle, un groupe $C_5$-$C_{12}$-cycloalkyle substitué par un groupe $C_1$-$C_4$-alkyle ou $C_1$-$C_4$-alcoxy ou un groupe phényle substitué par 1 à 3 groupes $C_1$-$C_4$-alkyle, $C_1$-$C_4$-alcoxy, $-SiR_4R_5R_6$, un halogène, $-SO_3M$, $-CO_2M$, $-PO_3M$, $-NR_7R_8$ ou $-[^+NR_7R_8R_9]X^-$; ou bien $R_2$ et $R_3$ sont différents et signifient un groupe $C_1$-$C_{12}$-alkyle, $C_5$-$C_{12}$-cycloalkyle, un groupe $C_5$-$C_{12}$-cycloalkyle substitué par un groupe $C_1$-$C_4$-alkyle ou $C_1$-$C_4$-alcoxy, un groupe phényle ou phényle substitué par 1 à 3 groupes $C_1$-$C_4$-alkyle, $C_1$-$C_4$-alcoxy, $-Si-R_4R_5R_6$, un halogène, $-SO_3M$, $-CO_2M$, $-PO_3M$, $-NR_7R_8$ ou $-[^+NR_7R_8R_9]X^-$; ou bien le groupe $-PR_2R_3$ représente un reste de formule II

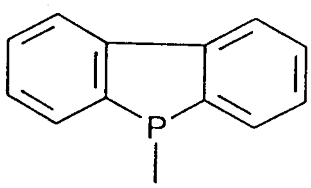

(II)

et $R_4$, $R_5$ et $R_6$ signifient indépendamment les uns des autres un groupe $C_1$-$C_{12}$-alkyle ou phényle, $R_7$ et $R_8$ signifient H, un groupe $C_1$-$C_{12}$-alkyle, phényle ou $R_7$ et $R_8$ représentent ensemble un groupe tétraméthylène, pentaméthylène ou 3-oxa-1,5-pentylène, $R_9$ signifie H ou un groupe $C_1$-$C_4$-alkyle, M signifie H ou un métal alcalin, $X^-$ signifie l'anion d'un acide monobasique et * caractérise un atome de carbone stéréogène, sous forme de leurs racémates et de leurs diastéréomères ou de mélanges de diastéréomères.

**20.** Tes complexes selon la revendication 19, caractérisés en ce qu'il s'agit de ceux de formule VI.

**21.** Tes complexes selon la revendication 19, caractérisés en ce que $X_1$ représente deux groupes éthylène, 1,5-hexadiène, 1,5-cyclooctadiène ou norbornadiène.

**22.** Les complexes selon la revendication 19, caractérisés en ce que Z signifie Cl ou Br.

**23.** Les complexes selon la revendication 19, caractérisés en ce que Z signifie $ClO_4^-$, $FSO_3^-$, $CH_3SO_3^-$, $CF_3SO_3^-$, $BF_4^-$, $PF_6^-$, $SbCl_6^-$, $AsF_6^-$ et $SbF_6^-$.

**24.** Les complexes selon la revendication 19, caractérisés en ce que $R_1$ en tant que groupe alkyle, est linéaire.

**25.** Les complexes selon la revendication 24, caractérisés en ce que $R_1$ signifie un groupe $C_1$-$C_4$-alkyle.

**26.** Tes complexes selon la revendication 25, caractérisés en ce que $R_1$ signifie un groupe méthyle ou éthyle.

**27.** Tes complexes selon la revendication 19, caractérisés en ce que $R_1$ signifie un groupe phényle ou phényle substitué par 1 ou 2 groupes $C_1$-$C_4$-alkyle ou $C_1$-$C_4$-alcoxy.

**28.** Les complexes selon la revendication 19, caractérisés en ce que $R_2$ et $R_3$ en tant que groupe alkyle sont linéaires ou ramifiés.

**29.** Tes complexes selon la revendication 28, caractérisés en ce que $R_2$ et $R_3$ signifient un groupe $C_1$-$C_8$-alkyle.

**30.** Tes complexes selon la revendication 28, caractérisés en ce que $R_2$ et $R_3$ sont identiques et signifient un groupe iso-propyle ou tert.-butyle.

**31.** Tes complexes selon la revendication 19, caractérisés en ce que $R_2$ et $R_3$ en tant que groupe cycloalkyle contiennent de 5 à 8 atomes de carbone dans le cycle.

**32.** Tes complexes selon la revendication 31, caractérisés en ce que $R_2$ et $R_3$ en tant que groupe cycloalkyle représentent un groupe cyclopentyle ou cyclohexyle.

**33.** Les complexes selon la revendication 19, caractérisés en ce que $R_2$ et $R_3$ en tant que groupe phényle substitué représentent un groupe 2-méthyl-, 3-méthyl-, 4-méthyl-, 2- ou 4-éthyl-, 2- ou 4-iso-propyl-, 2- ou 4-tert.-butyl-, 2-méthoxy-, 3-méthoxy, 4-méthoxy-, 2- ou 4-éthoxy-, 4-triméthylsilyl-, 2- ou 4-fluoro-, 2,4-difluoro-, 2- ou 4-chloro-, 2,4-dichloro-, 2,4-diméthyl-, 3,5-diméthyl-, 2-méthoxy-4-méthyl-, 3,5-diméthyl-4-méthoxy-, 3,5-diméthyl-4-(diméthylamino)-, 2- ou 4-amino-, 2-ou 4-méthylainino-, 2- ou 4-(diméthylamino)-, 2- ou 4-$SO_3H$-, 2- ou 4-$SO_3Na$-, 2- ou 4-[$^+NH_3Cl^-$]-, 2,4,6-triméthyl-phen-1-yle ou 3,4,5-triméthyl-phen-1-yle.

**34.** Les complexes selon la revendication 19, caractérisés en ce que $R_2$ et $R_3$ sont identiques et signifient un groupe cyclohexyle, 2- ou 4-méthylphen-1-yle, 2-ou 4-méthoxyphen-1-yle, 2- ou 4-(diméthylamino)phen-1-yle, 3,5-diméthyl-4-(diméthylamino)phen-1-yle et 3,5-diméthyl-4-méthoxyphen-1-yle.

**35.** Les complexes selon la revendication 19, caractérisés en ce que $R_2$ et $R_3$ sont différents et $R_2$ signifie un groupe phényle et $R_3$ signifie un groupe cyclohexyle, 2- ou 4-méthylphen-1-yle, 2- ou 4-méthoxyphen-1-yle, 4-(diméthylamino)phen-1-yle, 3,5-diméthyl-4-(diméthylamino)phen-1-yle, 3,5-diméthyl-4-méthoxyphen-1-yle ou 4-tert.-butylphen-1-yle.

**36.** Les complexes selon la revendication 19, caractérisés en ce que $R_2$ et $R_3$ représentent des restes identiques et signifient un groupe cyclohexyle.

**37.** Les complexes selon la revendication 19, caractérisés en ce que $R_2$ et $R_3$ représentent des restes identiques et signifient un groupe tert.-butyle ou o-anisyle.

**38.** Les complexes selon la revendication 19, caractérisés en ce que $R_2$ signifie un groupe phényle et $R_3$ signifie un groupe o-anisyle.

**39.** Les complexes selon la revendication 19, caractérisés en ce que $R_1$ signifie un groupe méthyle et $R_2$ et $R_3$ signifient chacun un groupe cyclohexyle.

**40.** L'utilisation des complexes des formules V et VI selon la revendication 19, comme catalyseurs homogènes pour l'hydrogénation asymétrique de composés pro-chiraux contenant des doubles liaisons de carbone ou de carbone/hétéroatome.

**41.** L'utilisation selon la revendication 40, pour l'hydrogénation de doubles liaisons de carbone dissymétriques, de cétimines et de cétones.

**42.** L'utilisation selon la revendication 40, pour l'hydrogénation de doubles liaisons de carbone dissymétriques avec des complexes de rhodium des formules V et VI.

**43.** Un procédé d'hydrogénation asymétrique de composés avec des doubles liaisons de carbone ou de carbone/hétéroatome sous des conditions de réaction homogènes, caractérisé en ce qu'on hydrogène les composés à une température de -20 à 80°C et sous une pression d'hydrogène de $10^4$ à $10^7$ Pa en présence de quantités catalytiques d'un complexe des formules V ou VI selon la revendication 19.

**44.** Un procédé selon la revendication 43, caractérisé en ce que la pression d'hydrogène est comprise entre $10^5$ et

$6{,}10^6$ Pa.

**45.** Un procédé selon la revendication 43, caractérisé en ce que la température est comprise entre - 10 et 50°C.

**46.** Un procédé selon la revendication 43, caractérisé en ce que la quantité du catalyseur est choisie afin que le rapport molaire du composé à hydrogéner (substrat) au complexe des formules V ou VI soit compris entre 10000 et 20.

**47.** Un procédé selon la revendication 43, caractérisé en ce que la réaction est effectuée en présence d'un solvant.

**48.** Un procédé selon la revendication 43, caractérisé en ce que le solvant est le méthanol ou un mélange de méthanol et de benzène ou de toluène.